# EUROPEAN PATENT APPLICATION

(11) **EP 2 145 678 A1**
(43) Date of publication of application: **20.01.2010**
(21) Application number: 09163189.5
(22) Date of filing: 19.06.2009
(51) Int. Cl.: B01J 23/06, B01J 21/06, B01J 35/02, C01G 9/02, C01G 23/00, C01G 23/047, C02F 1/32, A61L 9/20, F21V 3/04, F24F 3/12

(54) **TiO2-ZnO Nanocomposite film**

(30) Priority: 19.06.2008 TR 200804513
(71) Applicant: Durmus Yasar Ve Ogullari Boya Vernik Ve Recine Fabrikalari, 35210 Izmir (TR)
(72) Inventor: Demir, Hilmi Volkan, 06800 Ankara (TR); Celiker, Gulsen, 35620 Ankara (TR); Tek, Sumeyra, 06800 Ankara (TR); Sefunc, Mustafa Akin, 06800 Ankara (TR); Mutlugun, Evren, 06800 Ankara (TR); Yucel, Dilek, 35620 Izmir (TR)
(74) Representative: Dericioglu, Ekin

(57) **Abstract**

The present invention enables preparation and making a film of nano composites comprising TiO₂ and ZnO chemically integrated in one of the highly efficient acrylic/metacrylic, polyester, epoxy resins, and allows use of this material in biological cleaning due to its antibacterial feature and in chemical cleaning due to its feature of cleaning the pollution on the surfaces by degradation.
This invention additionally enables chemical and biological cleaning to be carried out by other lights such as the ones in the visible region in addition to the UV (ultra violet) light.

## Description

### Field of the Invention

The present invention relates to forming a film using photocatalytic nanomaterials and drawing this photocatalytic effect towards the visible region.

### Background of the Invention

We are facing biological and chemical pollutions as a problem both in daily life and in laboratory settings. One of the technological approaches for this for cleaning purposes is use of photocatalytic materials and coatings. Ultra violet (UV) light causes photocatalytic interaction on a surface including a photocatalyst. As a result of this photocatalytic interaction, electron-hole pairs are formed. Prior to combining of these carriers, photocatalytic interaction takes place by the successive reduction and oxidation reactions and cleaning takes place upon breaking of the chemical bonds of the organic impurities in the medium.

In the state of the art, titanium dioxide (TiO₂) and zinc oxide (ZnO) are used as a photocatalytic material in various areas such as in cleaning disk drives, cleaning refrigerators, eliminating odors, stain resistance, reducing the proportion of poisonous gases like NOx and COx in air and cleaning waters from wastes. Some metal oxide nanoparticles have been started to be used a lot recently due to their strong photocatalytic features. These materials are located in different architectures alone or as different layers in the same structure. Generally UV (Ultra violet) light is used for activating these materials. Photocatalytic activation of these materials is possible by their absorption of high energy photons from the band gap energy ranges. This limits the activation of wideband photocatalytic materials with the UV (Ultra violet) light spectrum.

TiO₂ is a photocatalytic material which has high oxidation effect when activated by ultra violet (UV) light. With this feature it enables degradation of undesired organics located on the surface. It can also be used in different implementations such as in attaining self cleaning surfaces and in cleaning air from nitrogen gas.

Many studies have been conducted in the state of the art for enhancing photocatalytic efficiencies of TiO₂ and ZnO. However there are no studies conducted related to preparing these two materials in the form of a single thin film and drawing the region where they are activated towards the visible region.

The Japanese patent document No. JP2005218957, a state of the art application, discloses a method developed for depositing a sulfur-added titanium dioxide film which is a very thin film having around several hundred nanometers thickness and exhibits a photocatalytic property in visible light. The visible light responsive titanium dioxide photocatalyst thin film is manufactured by using a target material prepared by firing titanium disulfide and adding sulfur to the target material as an impurity by a laser vapor deposition method.

The Greek patent document No. GR20050100138, one of the state of the art applications, discloses a chemical method for producing powders and thin films of nano-structured TiO₂ active to visible light. N, F, S and Se are added to the metal salt system at room temperature. Microadditives are added into the system in the gas phase at different temperatures, and powders and thin films of TiO₂ with nano-particles of various shapes and sizes active to visible light and having a strong photocatalytic and antibacterial action are prepared.

The Chinese patent document No. CN1785817, one of the state of the art applications, discloses a method for preparing nano ZnO/TiO₂ powder compounds capable of providing high protection against UV ray in UVA and UVB wavelengths. In the said method, metatitanic acid is used as titanium source while using zinc sulfate as zinc source, PEG 400 as surfactant, deionized water as solvent and ammonia water as precipitating agent to adjust the pH value. Ageing and drying are preformed to prepare Zn(OH)₂/TiO(OH)₂ metal salts and the said powder is calcined at high temperature so as to obtain nano ZnO/TiO₂ compounds.

The Japanese patent document No. JP2001070802, one of the state of the art applications, discloses preparation of a photocatalyst film improved in efficiency by increasing the porosity and specific surface area of the photocatalyst film. The specific surface area of this photocatalyst film can be increased compared with the conventional photocatalyst films. The photocatalyst film is comprised of compounds such as TiO₂, SrTiO₃, CdS, CdTe, Si, WO₃, MoS₂, Bi₂O₃, ZnO, SiC, GaP, GaAs.

The Chinese patent document No. CN1618518, one of the state of the art applications, discloses a composite TiO₂/ZnO photocatalyst used for controlling environmental pollution owing to the effect of photodegradation features of its carrier carbon nanotubes.

The Chinese patent document No. CN1626250, one of the state of the art applications, discloses preparation of a composition for cleaning air made up of activated bamboo carbon, nano material (TiO₂, ZnO, or SiO₂), modified starch, sodium dichloroisocyanurate, epoxy resin and a certain proportion of natural perfume. Its advantage is that it provides high and continuous photocatalytic disinfection.

The Canadian patent document No. CA2545638, one of the state of the art applications, discloses a process of producing a nano-TiO₂ aqueous emulsion with photocatalyst function. Filtered water soluble polyacrylate resin is loaded onto the nano catalyst. This photocatalyst coating can be applied to a wall, brick, leather, plastic, and wood by spray gun, brush or roller; and this coating strongly adheres objects which have photocatalytic activity under sunlight to the painted surface. In the production of this catalyst, nano zinc oxide is used to blend with nano TiO₂, which considerably enhances the photocatalytic function of the aqueous photocatalytic emulsion. It provides photocatalytic activity not only under ultraviolet radiation, but also in day light and under lamps in implementations in industry and daily life. It generates superoxide anions and hydroxyl radicals under ultraviolet radiation, in day light and under lamps, which decompose formaldehyde, toluene, organic solvents etc, meanwhile killing bacteria.

### Summary of the Invention

The present invention enables preparation and making a film of nano composites comprising TiO₂ and ZnO chemically integrated in one of the highly efficient acrylic/metacrylic, polyester, epoxy resins, and allows use of this material in biological cleaning due to its antibacterial feature and in chemical cleaning due to its feature of cleaning the pollution on the surfaces by degradation.

This invention enables chemical and biological cleaning to be carried out by other lights such as the ones in the visible region in addition to the UV (ultra violet) light.

### Detailed Description of the Invention

The experiment results of the thin films prepared to attain the objective of the present invention are illustrated in the accompanying figures, in which;
Figure 1 is the view of the measurement of the optical transmission of acrylic sol-gel film with TiO₂-ZnO nano composite integration stimulated by 330 nm monochromatic light.
Figure 2 is the view of the measurement of the optical transmission of acrylic sol-gel film with only TiO₂ nano particle integration stimulated by 330 nm monochromatic light.
Figure 3 is the view of the measurement of the optical transmission of acrylic sol-gel film with only ZnO nano particle integration stimulated by 330 nm monochromatic light.
Figure 4 is the view of the measurement of the optical transmission of acrylic sol-gel film without nano particles stimulated by 330 nm monochromatic light.
Figure 5 is the view of the % of optical recovery obtained from the analysis results given in Figure 1, 2, 3, 4 of the experiments which are carried out by stimulating in 16 different wavelengths from 310 nm to 469 nm.
Figure 6 is the view of the % of optical recovery of the film with acrylic sol-gel film without nanoparticles and TiO₂-ZnO nanocomposite film at stimulation wavelengths greater than 380 nm.
Figure 7 is the view of the measurement of the optical transmission of acrylic sol-gel film with TiO₂-ZnO nanocomposite integration stimulated by 403 nm monochromatic light.
Figure 8 is the view of the measurement of the optical transmission of acrylic sol-gel film with only TiO₂ nano particle integration stimulated by 403 nm monochromatic light.
Figure 9 is the view of the measurement of the optical transmission of acrylic sol-gel film with only ZnO nano particle integration stimulated by 403 nm monochromatic light
Figure 10 is the view of the measurement of the optical transmission of acrylic sol-gel film without nano particles stimulated by 403 nm monochromatic light

By using the inventive photocatalytic thin film with TiO₂-ZnO nanocomposite chemical integration, wide outdoor areas are enabled to be cleaned under sunlight or under other light sources such as visible region, by means of the photocatalytic synergy effect which light exhibits when stimulated by near ultra violet and visible region spectrum.

### Experiment

The characterization related to the experiments is based on the principle of measurement of the optical transmission spectroscopy in the process of optical activation of dry samples. Therefore, the dry samples are obtained via application of the wet samples to transparency sheet substrate. The product obtained as a result of chemical reaction is applied onto the transparency sheet with a thickness of 30 microns. This way, a thin film with the same form and same thickness is formed for all samples. For thinning the film; alcohol, ester, ketone, aromatic hydrocarbon solvent groups are used to attain the application viscosity of the nanocomposite. In the experiments, methylene blue is used which is one of the chemicals that are used as the standard pollutant. Samples which comprise no nano particles are prepared the same way for the negative control group. After optical transmission of the clean film is measured, it is polluted by the method of dropping methylene blue. Optical transmission of the film which is dried by nitrogen gun after waiting for 30 minutes is measured in polluted state. Later, optical transmission of the film which is stimulated at the desired wavelength for the desired period of time is measured again. Transmission measurements are conducted within the range of 450-750 nm. This range comprises the wavelengths at which the effect of methylene blue is observed. The optical transmission values decreasing due to the effect of the methylene blue approach to their original values as a result of the photocatalytic reactions. The ratio of the integral of the increase in these values to the area in between the clean and polluted transmission curves give the optical recovery value. These experiments are repeated for 16 different wavelengths of stimulation from 330 nm to 469 nm and for each of four films. The optical power and time are adjusted such that the number of photons per unit area on the film during the total stimulation period is fixed (10²² m⁻²). The samples are prepared so as to contain the same proportion of metal oxide (titanium dioxide, zinc oxide or both) nano particles in total. A considerable change (decrease in the transmission) is observed in the samples in the optical transmission spectroscopy in the visible region in dry samples before and after pollution (before optical activation). The specimens taken from each dry film after pollution are activated one by one at a certain optical wavelength and a certain optical power within the same area limits depending on time. Optical transmission spectroscopy measurements in reduced power are taken on these specimens at certain time intervals during activation and at the end of activation such that it will not affect activation in the visible region. This process is repeated at many different wavelengths from ultra violet to the visible region for comparing activation efficiency for each dry film. In these characterization processes, the polluting methylene blue starts to degrade (be cleaned) and its color fades during activation as a result of the photocatalytic activity, and this raises the optical transmission curve again and approaches it to its state before being polluted. At this point, in order to properly compare the measurements taken at different samples and wavelengths, optical activation and spectroscopy measurements are performed on the particle during activation process in the same period of time and same conditions such that the number of photons per unit area in unit time will be the same. The difference observed in the measurements arises from the different response of different dry films to different optical wavelengths (different photon energies). With these measurements, spectral photocatalytic response is attained for each kind of film as the function of the activation wavelength. As a result of the characterization, no optical cleaning (degradation of methylene blue) is observed at any of the wavelengths in the activation processes carried out the same way in control groups. This shows that methylene blue is not directly degraded (is not exposed to photohydrolysis) due to optical light used for self activation. For this reason, the changes observed in methylene blue in the other samples originate from the photocatalytic activities of metal oxides and are proportionate to the activity level.

It is found that the most active film as a result of the activation at deep ultra violet is, as expected, titanium dioxide, followed by titanium dioxide - zinc oxide combination and the last is zinc oxide. It is found that the most active film as a result of the activation at near ultra violet and visible region is titanium dioxide - zinc oxide combination and that this combination is better than titanium dioxide in the said spectrum range.

The inventive thin film and the acrylic sol-gel films with integration of only TiO₂ or only ZnO prepared in the same conditions in order for the effect of the inventive thin film to be understood, comprise nano materials that enable use of photocatalytic effect, which is strengthened by enhancing the active surface area by their fixation within the film separate from each other without aggregation, in cleaning environmental pollution in wide areas. In addition to the acrylic structure, resins selected from polyester and epoxy can also be used. The inventive film of TiO₂-ZnO nanoparticles integrated within acrylic sol-gel enables the photocatalytic degradation, which is limited by ultra violet stimulation for only TiO₂ or only ZnO, to continue substantially also when stimulated by the light spectrum of visible region. The acrylic sol-gel resin film not comprising nanoparticles is also prepared in control group experiments for testing purposes.

The inventive thin film comprises nanoparticles such that the ratio of TiO₂/ZnO is 0.33-3.00. The weight ratio of the total nanoparticles in their dispersions are maintained the same with the weight ratios in the dispersions in the films of only TiO₂ or only ZnO nanoparticles with chemical integration prepared for comparison purposes.

Sizes of the nanomaterials used are 2-20 nm for TiO₂ and 2-200 nm for ZnO. The reason of using anatase TiO₂ and ZnO metal oxides among many semi-conductors is that they have high photo-sensitivity, photo-stability and broad area of band gap energy. The fact that the speed of the re-combination of the electron - hole pair formed as a result of photo stimulation is low, causes these to diffuse to the surface and as a result of a series of reactions causes photocatalytic degradation.

Nano metal oxides dispersed in functional acrylic/metacrylic structure are hydrolyzed and during reaction of the monomers joining the reaction and hydrolyzed metal oxides with the reaction initiators, they are chemically integrated such that a three dimensional matrix is provided within the structure by covalent bonds by addition of functional alkoxy silanes. Bu using sol-gel method, the active photocatalytic nano material whose surface is coated with silane is prevented from degrading the resin into which it is integrated.

The obtained nanocomposite is thinned by alcohol, ester, ether, ketone, aromatic hydrocarbon solvent groups and brought to a film application viscosity.

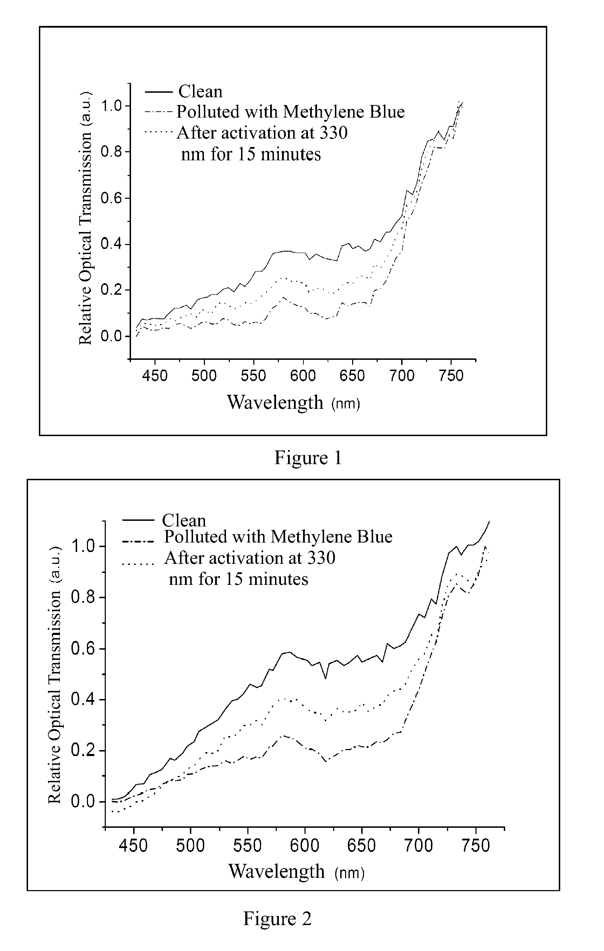

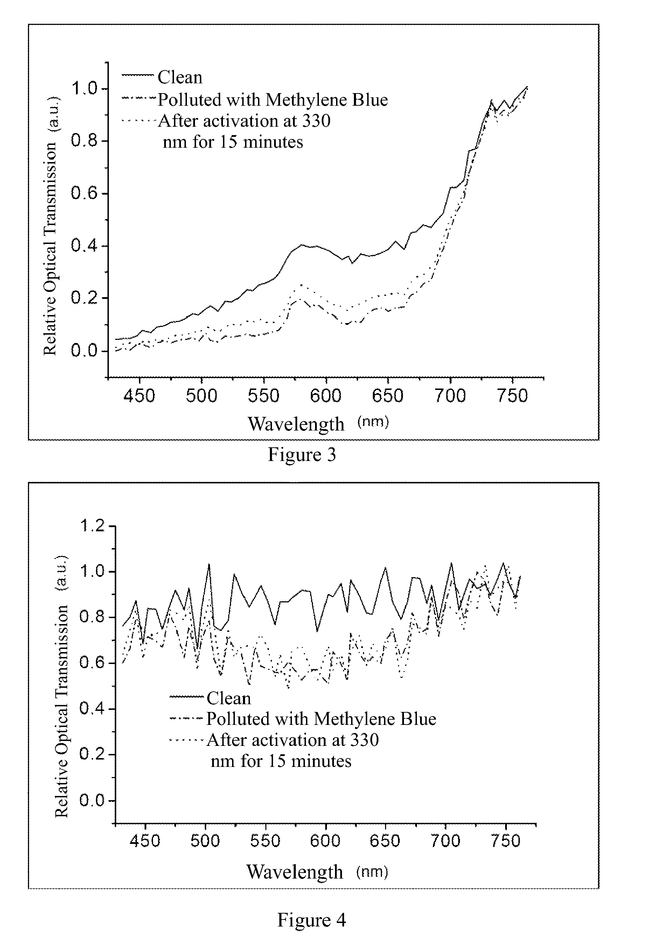

The inventive thin film which is TiO₂-ZnO nanocomposite within acrylic sol-gel exhibits less photocatalytic activity than TiO₂ and more photocatalytic activity than ZnO when stimulated at the ultra violet spectrum of light (Figure 1, 2, 3, 4, 5).

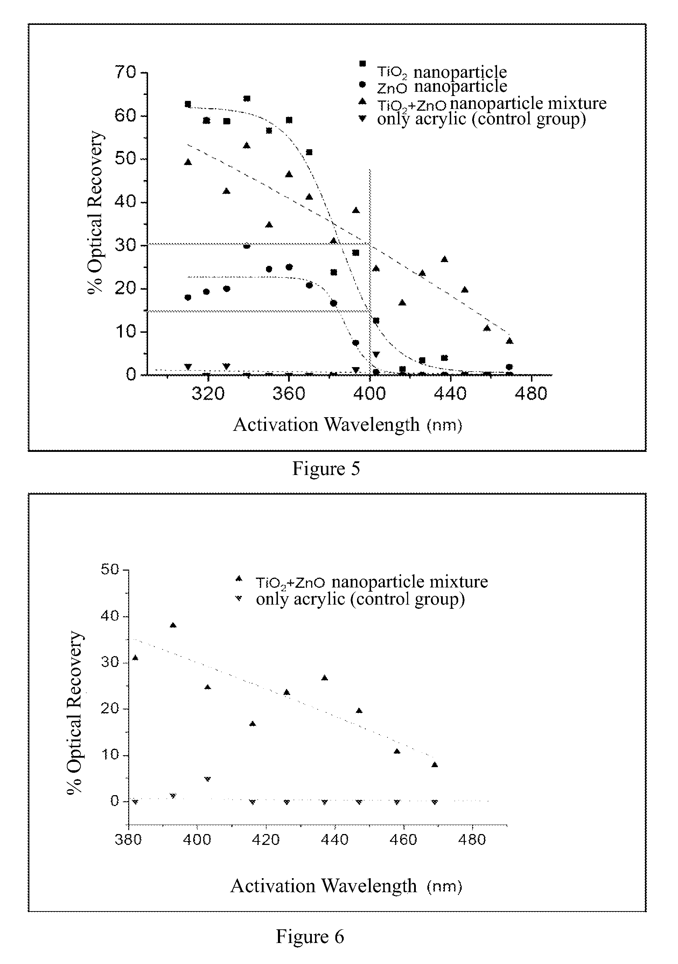

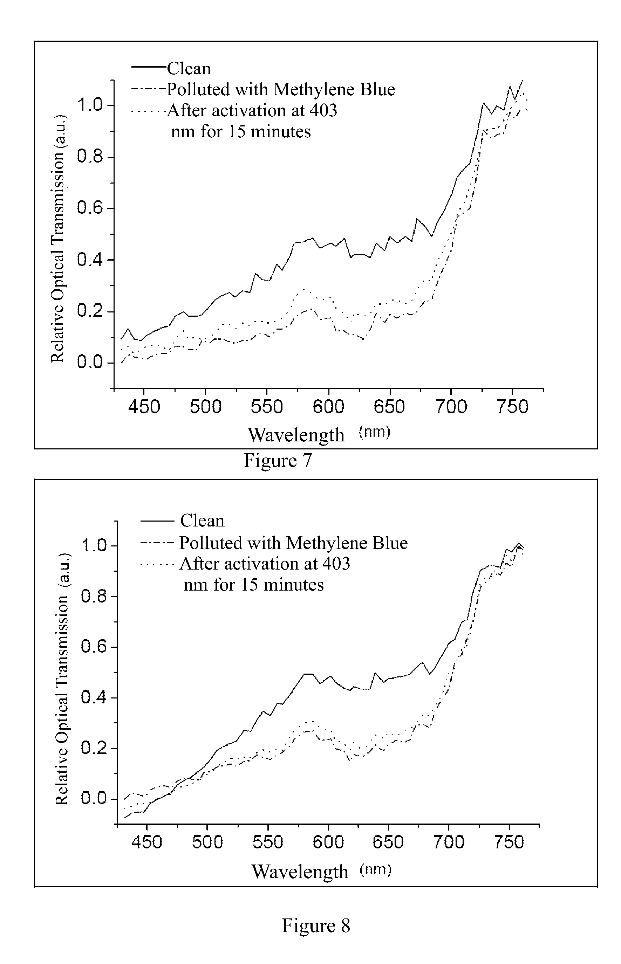

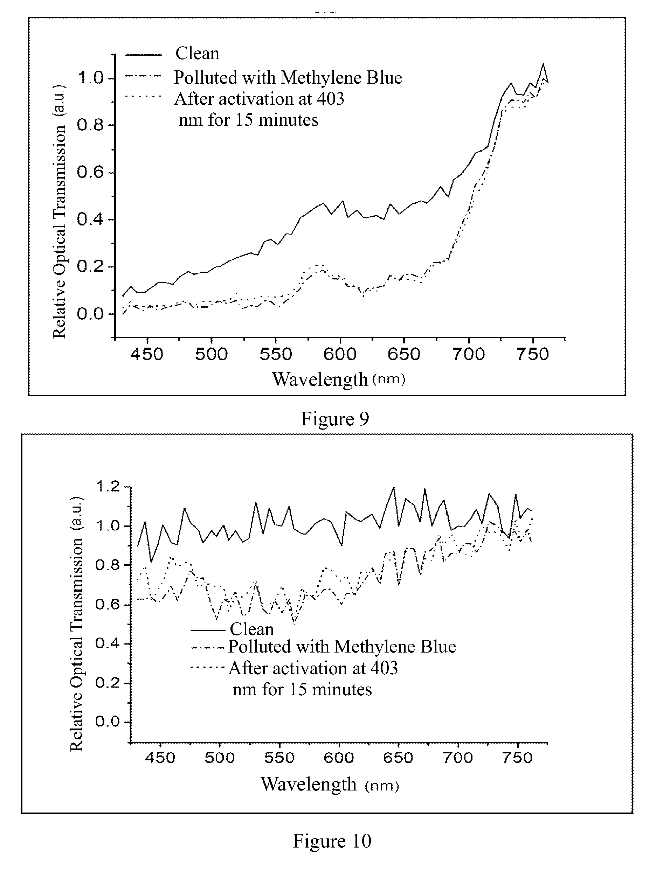

The band gap energy range for TiO₂ and ZnO is approximately 380 nm. Since the photons under this energy value can not be absorbed by metal-oxide, a significant decrease is observed in photocatalytic effect. It is observed that the inventive TiO₂-ZnO nanocomposite thin film has more photocatalytic activity at wavelengths of 380 nm and more, when compared to the only TiO₂ and only ZnO films. The inventive film has reached an optical recovery value of about 25-30 % even at visible light wavelengths at which photocatalytic activities of TiO₂ and ZnO approach to zero (Figure 6, 7, 8, 9, 10).

Optical recovery values of acrylic sol-gel thin film without nanoparticles is about zero at all experiments conducted at ultra violet and visible wavelengths. These experiments which are conducted with a control group demonstrate that the entire effect that we have measured originates from the photocatalytic degradation taking place in the presence of metal-oxide (Figure 5).

### Industrial Applicability of the Invention

The nanocomposite film is applied to the surface as a thin layer using one or more of the methods of spin coating, dipping, spray, roller, brush. The nanocomposite film obtained can be actively used in visible light in paints for inner and outer walls of houses, in electrical home appliances for cleaning the ambient air, in white goods, in environments where antimicrobial hygiene is required and in cleaning water.

The material can be used together with other material types such that a composite material will be formed again or can be used within mixtures. It becomes active in covered areas which do not have sunlight, such as tunnels, car parks, by the light emitted from lighting sources.

## Claims

1. A nanocomposite film which enables the stimulating wavelength to be drawn towards the visible region for chemical and biological cleaning and enables self cleaning under sunlight and other light sources within the visible region;
comprising the steps of
- hydrolyzing the nano metal oxides dispersed within the polymer structure,
- reacting the hydrolyzed metal oxides and monomers with the reaction initiators and enabling chemical integration of the nanoparticles into the three dimensional acrylic sol-gel with the addition of functional alkoxy silanes,
- coating the surface with silane by using sol-gel method to prevent degradation of the resin integrated within the active photocatalytic nanomaterial,
- thinning the nanocomposite by means of alcohol, ester, ether, ketone, aromatic hydrocarbon solvent groups,
- realizing optical characterization of nanocomposite films upon cleaning of them with the photocatalytic effect as a result of their activation with monochromatic wavelength, using different wavelengths between 330 - 469 nm as the stimulating wavelength.

2. A nanocomposite film according to Claim 1 **characterized in that** one of acrylic/metacrylic, polyester, epoxy is selected as the polymer.

3. A nanocomposite film according to Claim 1 and 2 **characterized by** using, among the nano metal oxides, titanium dioxide (TiO₂) and zinc oxide (ZnO) together which form synergy for the photocatalytic activity.

4. A nanocomposite film according to Claim 1 to 3 **characterized in that** size of the TiO₂ used is 2-20 nm, and size of the ZnO used is 2- 200 nm.

5. A nanocomposite film according to Claim 1 to 4 **characterized in that** the ratio of TiO₂/ZnO is 0.33 - 3.00.

6. A nanocomposite film according to Claim 1 to 5 **characterized in that** as a result of the activation at deep ultra violet, titanium dioxide is the most active, followed by titanium dioxide - zinc oxide combination and the last one is zinc oxide.

7. A nanocomposite film according to Claim 1 to 6 **characterized in that** titanium dioxide - zinc oxide combination is the most active as a result of the activation at near ultra violet and visible region.

8. A nanocomposite film according to Claim 1 to 7 **characterized in that** it is applied to the surface as a thin layer using one or more of the methods of spinning, dipping, spray, roller, brush.

9. A nanocomposite film according to Claim 1 to 8 **characterized in that** it is used together with other material types such that a composite material will be formed again or can be used within mixtures; in visible light in paints for inner and outer walls of houses, for cleaning the ambient air in electrical home appliances, in white goods, in environments where antimicrobial hygiene is required, in cleaning water; under lights emitted by lighting sources at environments like tunnels, car parks where sunlight is not visible.
